# EUROPÄISCHE PATENTSCHRIFT

(11) **EP 1 491 170 B1**
(45) Veröffentlichungstag und Bekanntmachung des Hinweises auf die Patenterteilung: **14.09.2005**
(21) Anmeldenummer: 04014082.4
(22) Anmeldetag: 16.06.2004
(51) Int. Cl.: A61F 5/44, B29C 41/14

(54) **Urinalkondom und Verfahren zu dessen Herstellung**
Urinary condom and manufacturing method
Préservatif urinaire et son procédé de fabrication

(30) Priorität: 27.06.2003 DE 10329129
(43) Veröffentlichungstag der Anmeldung: 29.12.2004
(73) Patentinhaber: Willy Rüsch GmbH, 71394 Kernen-Rommelshausen (DE)
(72) Erfinder: Kepp, Werner, 71323 Waiblingen (DE)
(74) Vertreter: Kohler Schmid Möbus

(56) Entgegenhaltungen:
- EP-A- 0 147 072
- WO-A-91/17728
- WO-A-02/053070
- DE-A- 3 236 396
- US-A- 5 014 361

## Beschreibung

Die Erfindung betrifft ein latexfreies Urinalkondom für männliche Patienten, insbesondere einen Außenkatheter zum Auffangen und Ableiten unwillkürlicher Urinentleerungen und ein Verfahren zur Herstellung des Urinalkondoms. Derartige Urinalkondome weisen ein kondomartiges Hüllenteil und eine verstärkte Spitze, bestehend aus einem zylinderförmigen Katheterteil und einem konischen Zwischenstück zum Verbinden der Spitze mit dem Hüllenteil, auf.

In der Patentschrift DE 3236396A wird ein Urinalkondom offenbart, das jedoch nicht latexfrei ist. Der Hüllenteil des Urinalkondoms ist aus einer Latexschicht, einer Klebstoffschicht und einer Trennschicht aus Silikonkautschuk zwischen der Latexschicht und der Klebstoffschicht aufgebaut. Der Hüllenteil ist in Richtung der Spitze derart aufgerollt, dass die Klebstoffschicht zwischen der Trennschicht und der Basisschicht eingerollt ist, wobei die Klebstoffschicht stärker an der Basisschicht als an der Trennschicht anhaftet. Wenn der aufgerollte Hüllenteil des Urinalkondoms über den Penis des Urinalkondombenutzers abgerollt wird, bleibt die Klebeschicht an der Basisschicht anhaften und damit wird die Basisschicht an der Penishaut angeklebt. Nachteilig an dem Urinalkondom ist, dass es sich bei Latex um ein Material handelt, auf das viele Menschen allergisch reagieren. Weiterhin hat Latex eine geringe Feuchtigkeitsdurchlässigkeit und Urinalkondome aus Latex müssen mit relativ großen Wandstärken des Hüllenteils gefertigt sein, was die Feuchtigkeitsdurchlässigkeit des Urinalkondoms weiter vermindert. Diese geringe Feuchtigkeitsdurchlässigkeit führt bei Langzeitanwendungen zu Hautirritationen, Entzündungen und ähnlichen Hautproblemen. Zudem sind Urinalkondome aus Latex wegen ihrer dicken Wandstärke undurchsichtig, wodurch die erwähnten Probleme nicht frühzeitig diagnostiziert werden können.

Die Offenlegungsschrift WO 96129963 offenbart ein Urinalkondom mit einem Hüllenteil aus einer Polyurethanschicht und dessen Herstellung aus einem Polyurethan in einem flüssigen Zustand. Bei diesem Polyurethan in einem flüssigen Zustand handelt es sich um das Prepolymer von Polyurethan, welches durch Erwärmen zu einem Polyurethan vernetzt wird. Zur Verbesserung der Verarbeitungseigenschaften werden Verarbeitungszusätze, d.h. Chemikalien wie Emulgatoren, Verdickungs- und Netzmittel eingesetzt, welche auch noch im Urinalkondom vorhanden sind und zu Unverträglichkeiten führen können. Die Herstellung des Urinalkondoms erfolgt im Tauchverfahren. Die Tauchform wird in ein Bad aus Prepolymer eingetaucht und mit einer definierten Geschwindigkeit wieder aus dem Prepolymerbad herausgezogen. Die aufgetauchte Prepolymerschicht wird innerhalb von 40 Minuten bei schrittweiser Temperaturerhöhung von 60 bis 190 Grad Celsius zu Polyurethan vernetzt. Nach Abkühlen auf 60 Grad Celsius wird der Beschichtungsvorgang so lange wiederholt, bis eine gewünschte Schichtdicke erreicht ist. Die verstärkte Spitze wird ebenfalls im Tauchverfahren, allerdings mit einer unterschiedlichen Tauchbadmischung als die für den dünnwandigen Hüllenteil, hergestellt. Der Herstellungsprozess des offenbarten Urinalkondoms ist damit sehr aufwendig und erfordert eine zeit- und kostenintensive Vernetzung bei hohen Temperaturen.

Die WO 96/08353 offenbart ein Herstellungsverfahren von elastischen Artikeln, zum Beispiel von Kondomen, die eine Polyurethanschicht aufweisen. Die Artikel werden unter Verwendung eines Tauchverfahrens aus einer wässrigen Dispersion eines Polyurethans, die eine anionische Stabilisierung aufweist, hergestellt. Nachteilig bei diesem Verfahren ist, dass in der Dispersion Zusätze, z.B. ein Weichmacher und eine oberflächenaktive Substanz, nämlich ein anionischer Stabilisator, enthalten sind. Diese Zusätze verbleiben nach der Herstellung in der Polyurethanschicht. Die Zusätze können, da sie nicht fest in die Molekülstruktur der Polyurethanschicht eingebaut sind, während einer Benutzung der Artikel entweichen, z.B. ausdampfen, wodurch Hautunverträglichkeiten auftreten können. Vermeidbar wäre dieser Nachteil durch ein Auswaschen des fertigen Artikels in einem Wasserbad. Dies würde jedoch zu einer erheblichen Produktionskostensteigerung führen.

Der Erfindung liegt die Aufgabe zugrunde, ein latexfreies Urinalkondom und ein Verfahren zu dessen Herstellung bereitzustellen, welche die Nachteile des Standes der Technik vermeidet und insbesondere einfach, schnell und kostengünstig herzustellen und unter weitgehender Vermeidung von Hautunverträglichkeiten anzuwenden ist.

Diese Aufgabe wird durch das Verfahren und die Vorrichtung der unabhängigen Ansprüche gelöst. Die abhängigen Ansprüche stellen bevorzugte Ausführungsformen der Erfindung dar.

Erfindungsgemäß wird die Aufgabe dadurch gelöst, dass ein Urinalkondom mit einer verstärkten Spitze und einem Hüllenteil zur Verfügung gestellt wird, wobei das Urinalkondom mindestens eine Basisschicht aus Polyurethan aufweist und die Polyurethanschicht aus einer wässrigen Dispersion eines aliphatischen Polyurethans, die eine anionische Stabilisierung aufweist, hergestellt ist. Erfindungsgemäß ist in die Molekülketten des Polyurethans ein anionischer Stabilisator, bevorzugt ein anionisches Tensid, zur anionischen Stabilisierung der wässrigen Dispersion, eingebaut. Unter aliphatisch ist eine organische Verbindung mit offenen Kohlenstoffketten in deren Strukturformel zu verstehen. Derartige Dispersionen sind anionisch stabilisiert, wodurch keine Emulgatoren verwendet werden müssen. Im Gegensatz zum Stand der Technik muss die Dispersion daher keinen frei gelösten anionischen Stabilisator enthalten. Bei dem Polyurethan handelt es sich um ein aliphatisches Polyurethan, in dessen Molekülketten ein anionischer Stabilisator, bevorzugt ein anionisches Tensid, zur anionischen Stabilisierung der wässrigen Dispersion, eingebaut ist, wodurch die anionischen Gruppen, also der Stabilisator, fest im Polyurethanpolymer der Polyurethanschicht des Urinalkondoms eingebaut sind und nicht bei einer Verwendung des erfindungsgemäßen Urinalkondoms entweichen können.
Die verstärkte Spitze besteht dabei üblicherweise aus einem konischen Teil, der sich an das Hüllenteil anschließt. Am verjüngten Ende des konischen Teils ist ein zylinderförmiger Katheterteil, also ein flexibles Rohr bzw. schlauchartiger Abschnitt, vorgesehen, durch welches der Harn ablaufen kann.
Ein wesentlicher Vorteil der erfindungsgemäß verwendeten Dispersion ist es, dass sie frei von Emulgatoren und frei gelösten anionischen Stabilisatoren ist, wodurch auch die Polyurethanschicht des Urinalkondoms frei von Rückständen derartiger Chemikalien ist. Das erfindungsgemäße Urinalkondom ist dadurch sehr gut hautverträglich. Eine Zugabe von Verarbeitungshilfsmitteln oder Vernetzern ist nicht erforderlich. Ein Auswaschen des Urinalkondoms bei der Herstellung kann unterbleiben. Ferner weist die Polyurethanschicht des erfindungsgemäßen Urinalkondoms eine günstige Beschichtungsfähigkeit auf. Eine aufgebrachte Beschichtung kann nicht mit entweichenden Chemikalien, insbesondere Stabilisatoren, chemisch reagieren und/oder durch die entweichenden Chemikalien abgelöst werden, beziehungsweise erst gar nicht anhaften.

Bevorzugt ist die Dispersion und damit die Polyurethanschicht des Urinalkondoms frei von Weichmachern. Weichmacher enthaltende Polyurethane sind besonders ungeeignet zur Verwendung zur Herstellung von Urinalkondomen, da diese häufig über längere Zeitabschnitte mit der Haut von Patienten in Kontakt kommen und damit bekannte Unverträglichkeiten von Weichmachern besonders stark auftreten.

Das erfindungsgemäße Urinalkondom aus Polyurethan weist eine sehr geringe Wandstärke bis maximal 0,15 mm auf. Die Wandstärke der Urinalkondome aus anderen Materialien liegt dagegen bei 0,25 bis 0,3 mm. Dies ermöglicht eine bessere Anpassung an die Haut sowie ein sichereres Abdichten im Falle einer Faltenbildung. Weiter hat das erfindungsgemäße Urinalkondom aus Polyurethan eine wesentlich höhere Wasserdampfdurchlässigkeit als die Urinalkondome aus Silikon, Latex oder einem thermoplastischen Elastomer (TPE). Urinalkondome aus Polyurethan sind durchsichtig und ermöglichen bei angelegtem Urinalkondom eine Inspektion der Haut.
Das erfindungsgemäße Urinalkondom wird durch Tauchen in eine emulgatorfreie Dispersion hergestellt. Eine Vernetzung eines Prepolymers zu Polyurethan ist damit nicht notwendig. Es wird ausschließlich eine Trocknung durchgeführt.
Durch Verwenden einer wässrigen Polyurethandispersion wird bei dem Herstellen des Urinalkondoms erheblich Herstellungszeit eingespart. Das erfindungsgemäße Urinalkondom kann in einem einzigen Tauchschritt hergestellt werden. Die Anzahl der Tauchschritte wird nicht von der gewünschten Wandstärke des Urinalkondoms bestimmt, sondern durch weitere Qualitätsanforderungen, wie z.B. der maximal tolerierten Ausschussrate.

Bevorzugt weist die Spitze eine Verstärkungsschicht auf, wobei die Verstärkungsschicht durch Verwenden eines Koagulants, also eines ein Ausflocken des Polyurethans aus der Dispersion bewirkenden Mittels, bevorzugt einer Calciumnitratlösung aus der wässrigen Dispersion des aliphatischen Polyurethans, hergestellt ist. Die Verstärkungsschicht kann dabei sowohl auf die Basisschicht aufgebracht sein, als auch sich an die Basisschicht des Hüllenteils anschließen. Das Herstellen der verstärkten Spitze mit Hilfe eines Koagulants hat den Vorteil, dass das beim Aufbringen der Dispersion zum Ausbilden der Spitze eine gegenüber dem Nichtverwenden eines Koagulants verdickte Dispersionsschicht in einem Arbeitsgang aufgebracht werden kann. Dies verkürzt die Herstellungszeit des Urinalkondoms signifikant.

Der Hüllenteil des erfindungsgemäßen Urinalkondoms weist bevorzugt eine Wandstärke von 0,05 bis 0,1 mm auf. Die Spitze weist bevorzugt eine Wandstärke von 0,1 bis 0,5 mm auf. Diese Wandstärken verbinden vorteilhaft eine gute Wasserdampfdurchlässigkeit mit einer ausreichenden mechanischen Stabilität des Urinalkondoms.

In einer bevorzugten aufgerollten Ausführungsform weist der Hüllenteil des Urinalkondoms mindestens eine klebstoffabweisende (antiadhäsive) Trennschicht und eine Klebstoffschicht, bevorzugt aus Acrylatklebstoff, auf. Dabei ist die Trennschicht zwischen der Basisschicht und der Klebstoffschicht angeordnet. Der Hüllenteil des Urinalkondoms ist in Richtung der Spitze derart aufgerollt ist, dass die Klebstoffschicht zwischen der Trennschicht und der Basisschicht eingerollt ist, wobei die Klebstoffschicht stärker an der Basisschicht als an der Trennschicht anhaftet. Diese Ausführungsform hat den Vorteil, dass nach Abrollen des Urinalkondoms bei dessen Anwendung der Klebstoff an der Innenseite der Basisschicht aus Polyurethan anhaften bleibt und damit ein Verkleben der Basisschicht des Urinalkondoms mit der Haut des Penis, auf welchen das Urinalkondom aufgerollt wird, gewährleistet. Dadurch wird eine einfache Handhabung und eine sichere Applikation des Urinalkondoms ermöglicht.

Die Trennschicht des Urinalkondoms besteht bevorzugt aus einem Silikonkautschuk, wiederum bevorzugt einem Silikonkautschuk mit einer Vernetzungstemperatur zwischen 50 und 100 Grad Celsius. Dies hat den Vorteil, dass Silikon gute antiadhäsive Eigenschaften hat und bei den niedrigen Vernetzungstemperaturen keine vollständige Austrocknung der Basisschicht gewährleistet sein muss.

Hinsichtlich des Verfahrens wird die Aufgabe dadurch gelöst, dass zum Herstellen des Urinalkondoms beim Ausbilden der Basisschicht und/oder der verstärkten Spitze ein Tauchverfahren verwendet wird. Dieses Tauchverfahren hat die Tauchverfahrensschrittfolge:
(a) Bereitstellen eines Tauchbades aus einer wässrigen Dispersion eines aliphatischen Polyurethans, die eine anionische Stabilisierung aufweist, wobei in die Molekülketten des Polyurethans ein anionischer Stabilisator, bevorzugt ein anionisches Tensid, zur anionische Stabilisierung der wässrigen Dispersion, eingebaut ist,
(b) Eintauchen einer der gewünschten Urinalform entsprechenden Tauchform in das Tauchbad,
(c) Herausziehen der Tauchform aus dem Tauchbad, wobei an der Tauchform eine Schicht der Dispersion anhaften bleibt und
(d) Ausbilden einer Polyurethanschicht durch Trocknen der an der Tauchform anhaftenden Dispersion.

Die Tauchverfahrensschrittfolge wird mindestens einmal, im Falle des Ausbildens des Hüllenteils bevorzugt zweimal durchgeführt.
Aufgrund des erfindungsgemäßen Verwendens einer wässrigen Dispersion ist keine Vernetzung nach dem Tauchprozess notwendig, sondern lediglich eine Trocknung. Dies führt zu einer Verfahrensbeschleunigung.

Zum Herstellen der verstärkten Spitze wird bevorzugt ein Koagulant, bevorzugt eine Calciumnitratlösung, im Bereich der Spitze des Urinalkondoms auf die Tauchform vor dem Durchführen einer Tauchverfahrensschrittfolge aufgebracht. Dabei kann das Koagulant entweder direkt vor einer ersten Tauchverfahrensschrittfolge im Bereich der Spitze auf die Tauchform aufgebracht werden oder das Koagulant wird vor einer weiteren Tauchverfahrensschrittfolge auf die Basisschicht im Bereich der Spitze des Urinalkondoms auf der Tauchform aufgebracht. Nach dem Herausziehen der Tauchform aus dem Tauchbad haftet auf der Basisschicht oder auf der Tauchform eine durch das Koagulant erzeugte verstärkte Dispersionsschicht an. Die Verstärkungsschicht bzw. die verstärkte Spitze wird wiederum durch Trocknen der anhaftenden Dispersion ausgebildet.

Das Koagulant wird bevorzugt durch Tauchen des Bereichs der Spitze des Urinalkondoms, bzw. des Bereichs der Spitze der Tauchform in ein Tauchbad aus Koagulantlösung aufgebracht. Die Polyurethandispersion wird mittels eines Koagulant-Tauchverfahrens verarbeitet, wodurch auch für die verstärkte Spitze nur ein Tauchschritt in der Polyurethandispersion ausreicht. Gemäß dem vorbekannten Stand der Technik sind für die verstärkte Spitze mindestens sechs bis sieben Tauchschritte erforderlich.

Bevorzugt wird nach Abschluss der Tauchverfahrensschrittfolge oder Tauchverfahrensschrittfolgen eine Trennschicht, bevorzugt aus Silikonkautschuk, auf die Polyurethanschicht des Hüllenteils des Urinalkondoms und auf die Trennschicht eine Klebstoffschicht, bevorzugt durch Sprühnebelauftragen, aufgebracht. Die Trennschicht wird bevorzugt durch Tauchen des Hüllenteils der Polyurethanschicht auf der Tauchform in ein Silikonkautschukbad oder durch Aufbringen einer Sprühnebelkautschukschicht auf den Hüllenteil der Polyurethanschicht auf die Tauchform aufgebracht. Zum Ausbilden der Trennschicht wird bevorzugt ein Silikonkautschuk mit einer Vernetzungstemperatur zwischen 50 und 100 Grad Celsius verwendet. Der Sprühnebelauftrag hat den Vorteil, dass keine aufwendige Tauchanlage für die Silikonschicht und/oder den Klebstoff bereit gestellt werden muss.

Das erfindungsgemäß hergestellte Urinalkondom wird bevorzugt durch Aufrollen des Hüllenteils des Urinalkondoms in Richtung der Spitze von der Tauchform abgenommen. Hierdurch wird gleichzeitig mit dem Abnehmen von der Tauchform die bevorzugte aufgerollte Ausführungsform des Urinalkondoms hergestellt.

Beim Anwenden der bevorzugten aufgerollten Ausführungsform des Urinalkondoms wird der aufgerollte Hüllenteil des Urinalkondoms über den Penis des Urinalkondombenutzers abgerollt, wobei die Klebeschicht an der Basisschicht anhaften bleibt und damit die Basisschicht an der Penishaut angeklebt wird.

Weitere Vorteile ergeben sich aus der Beschreibung und den beigefügten Zeichnungen. Die vorstehend genannten und die noch weiter aufgeführten Merkmale der Erfindung können jeweils einzeln oder in Kombination miteinander verwendet werden. Die erwähnten Ausführungsformen sind nicht als abschliessende Aufzählung zu verstehen, sondern haben vielmehr beispielhaften Charakter.

Die Erfindung wird nachfolgend anhand von Ausführungsbeispielen unter Bezugnahme auf die Zeichnungen näher erläutert.
Fig. 1 zeigt ein erfindungsgemäßes Urinalkondom bei dessen Anwendung;
Fig. 2 zeigt mit den Fig. 2a und 2b ein erfindungsgemäßes Urinalkondom in einer bevorzugten Ausführung in aufgerolltem Zustand;
Fig. 3 zeigt ein Ablaufdiagramm des Verfahrens zum Herstellen eines erfindungsgemäßen Urinalkondoms: und
Fig. 4 zeigt eine Tabelle bezüglich der Wasserdampfdurchlässigkeit von Urinalkondomen aus verschiedenen Materialien.

Die Fign. der Zeichnungen zeigen den erfindungsgemäßen Gegenstand stark schematisiert und sind nicht massstäblich zu verstehen. Die einzelnen Bestandteile des erfindungsgemäßen Gegenstandes sind so dargestellt, dass ihr Aufbau gut gezeigt werden kann.

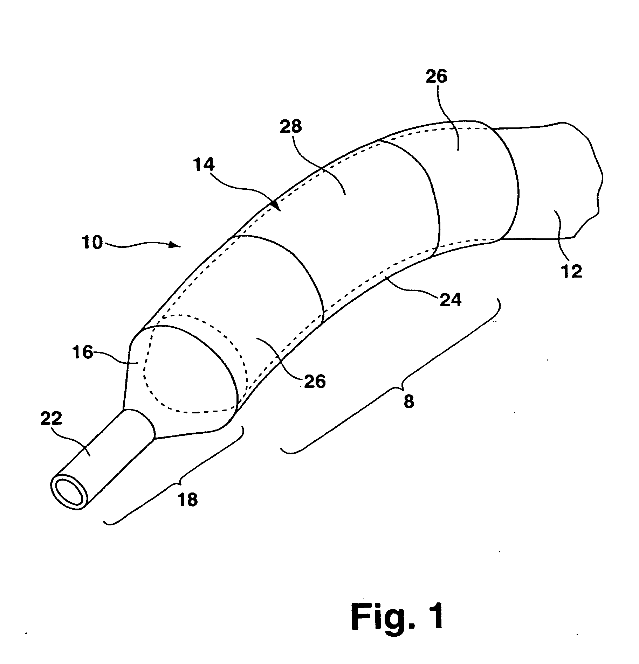

In Fig. 1 wird das erfindungsgemäße selbstklebende Urinalkondom 10 bei dessen Anwendung, also nach Abrollen des Hüllenteils 8 über den Penis 12 des Urinalkondombenutzers dargestellt. In der Figur ist die Schichtfolge des Hüllenteils 8 zu erkennen. Die verstärkte Spitze 18 des Urinalkondoms 10 besteht aus einem Rohr 22 und einem konischen Teil 16. Der Hüllenteil 8 ist im auf den Penis 12 aufgebrachten Zustand, wie dargestellt, aus der Schichtfolge: Klebstoffschicht 24, Polyurethanbasisschicht 26 und Trennschicht 28 aufgebaut. In der Figur ist in einem Mittelbereich des Hüllenteils die Polyurethanbasisschicht durch zwei begrenzende Linien stark schematisiert dargestellt, so dass die Trennschicht freigelegt ist. Bei der Herstellung des Urinalkondoms 10 wurde die Klebstoffschicht 24 auf der Trennschicht 28 aufgebracht. Erst durch das Abrollen bei der Anwendung wird die Klebstoffschicht 24 zwischen Penishaut und Basisschicht 26 verbracht, da der Klebstoff stärker an der Basisschichtinnenseite 14 als an der Trennschicht 28 anhaftet.
Die Außenseite von selbstklebenden Urinalkondomen muss klebstoffabweisend (antiadhäsiv) sein um das aufgerollte Urinalkondom wieder abrollen zu können. Dies wird durch die antiathäsive Trennschicht, welche auf die Basisschicht 26 aus Polyurethan aufgebracht ist, erreicht. Die Trennschicht 28 besteht aus einem Silikonkautschuk.
Bevorzugt handelt es sich dabei um einen Silikonkautschuk, welcher bei Temperaturen deutlich unter 100 Grad Celsius innerhalb weniger Sekunden vernetzt, z.B. in 4,6 s bei 80 Grad Celsius. Diese niedrige Vernetzungstemperatur und die kurze Vernetzungszeit sind sehr vorteilhaft für einen effizienten Herstellungsprozess. Prinzipiell kann ein bei Raumtemperatur vernetzendes Silikon verwendet werden. Dies führt jedoch zu langen Vernetzungszeiten. Ein Verwenden von Silikonkautschuk, welcher bei über 100 Grad Celsius vernetzt, hat den Nachteil, dass vor dessen Aufbringen die Polyurethanschicht vollständig durchgetrocknet sein muss, was zu sehr langen Trocknungszeiten führt.

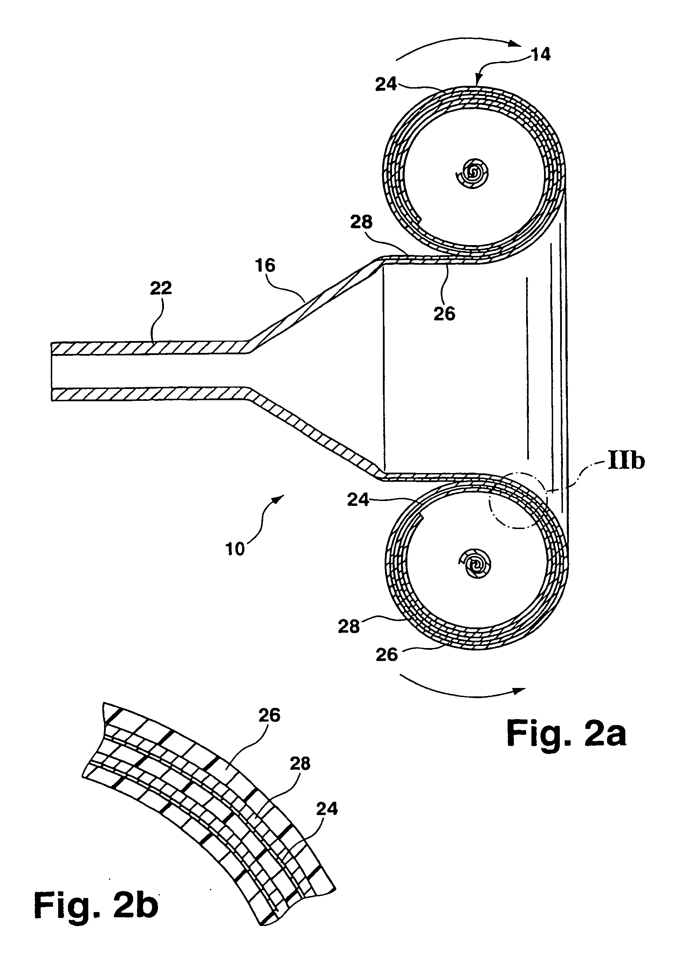

In Fig. 2 wird mit den Fig. 2a und 2b die bevorzugte aufgerollte Ausführungsform des erfindungsgemäßen selbstklebenden Urinalkondoms dargestellt. Das Hüllenteil des Urinalkondoms ist aus einer Basisschicht 26 aus Polyurethan, die aus einer wässrigen Dispersion eines aliphatischen Polyurethans hergestellt ist, einer klebstoffabweisenden Trennschicht 28 und einer Klebstoffschicht 24 aufgebaut. Die klebstoffabweisende Trennschicht ist aus einem Silikon, das bei einer Temperatur von unterhalb 100 Grad Celsius eine schnelle Vernetzung garantiert, hergestellt. Die Basisschicht 26 hat eine innere Oberfläche 14 und eine äußere Oberfläche. Die klebstoffabweisende Trennschicht 28 ist auf der äußeren Oberfläche der Basisschicht 26 aufgebracht. Zusätzlich ist auf der klebstoffabweisenden Schicht 28 eine Klebstoffschicht 24 aufgebracht, die im aufgerollten Zustand des Urinalkondoms von der inneren Oberfläche 14 der Basisschicht 26 abgedeckt ist, so dass die im aufgerollten Zustand des Urinalkondoms freiliegenden Flächen keine klebenden Eigenschaften aufweisen. Wird das Urinalkondom abgerollt, so greift die innere Oberfläche 14 der Basisschicht 26 die auf der klebstoffabweisenden Trennschicht 28 liegende Klebeschicht 24 auf, weil sie stärker an der Klebeschicht 24 anhaftet und nimmt diese Klebeschicht 24 vollkommen mit, so dass im insgesamt abgerollten Bereich des Urinalkondoms nunmehr die Klebeschicht 24 an der inneren Oberfläche 14 der Basisschicht 26 ausgebildet ist. Damit ist die innere Oberfläche 14 der Basisschicht 26 im abgerollten Zustand, also bei der Anwendung des Urinalkondoms, an die Haut des Penis angeklebt. Die Außenseite von selbstklebenden Urinalkondomen muss klebstoffabweisend (antiadhäsiv) sein, um das aufgerollte Urinalkondom wieder abrollen zu können. Dies wird durch die antiadhäsive Trennschicht, welche auf die Basisschicht aus Polyurethan aufgebracht ist, erreicht. Die Trennschicht 28 besteht aus einem Silikonkautschuk, bevorzugt mit einer Vernetzungstemperatur unter 100 Grad Celsius.

Fig. 2b zeigt eine vergrößerte Darstellung der Schichtenfolgen an IIb aus Fig. 2a.

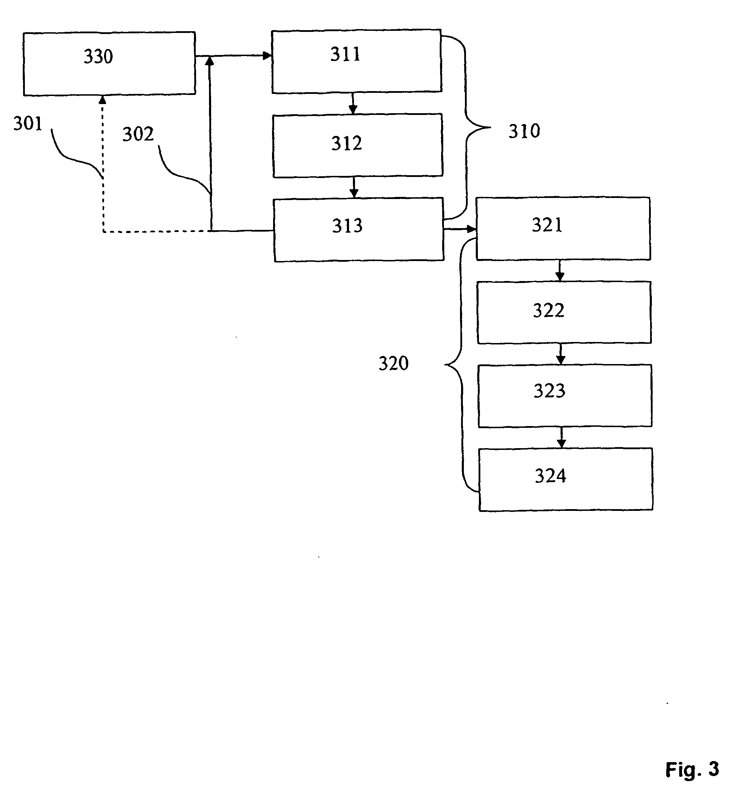

In Fig. 3 wird die Herstellung des erfindungsgemäßen Urinalkondoms mittels einer wässrigen Dispersion eines aliphatischen Polyurethans in einem Ablaufdiagramm dargestellt. Die Herstellung erfolgt mittels eines Tauchverfahrens. Dabei wird eine Tauchverfahrensschrittfolge 310, welche aus den Schritten Eintauchen einer der gewünschten Urinalkondomform entsprechenden Tauchform in ein bereit gestelltes Tauchbad aus der Dispersion 311, Herausziehen der Tauchform 312 aus dem Tauchbad und Ausbilden der Polyurethanschicht durch Trocknen 313 besteht. Die aufgetauchten Schichten (Tauchschicht) der Dispersion werden also lediglich getrocknet. Dies kann prinzipiell bei Raumtemperatur erfolgen, bevorzugt wird bei einer Temperatur von 40 bis 60 Grad Celsius getrocknet. Bei diesen Temperaturen ist ein ausreichend vollständiges Trocknen des Tauchfilms in weniger als sechs Minuten möglich.

Die Herstellung der verstärkten Spitze wird durch Verwenden eines Koagulants, bevorzugt einer Calciumnitratlösung, durchgeführt. Vor dem Eintauchen in die Polyurethandispersion wird der Spitzenbereich der Tauchform mit Koagulant betaucht, d.h. es wird ein Koagulant im Bereich der Spitze aufgebracht (Bezugszeichen 330). Beim nachfolgenden Tauchen in die Polyurethandispersion wird durch das Koagulant auf dem Spitzenbereich eine gleichmäßig dicke Schicht auf der Tauchform abgeschieden. Die Dicke der Schicht, bevorzugt maximal 0,5 mm, wird durch die Verweildauer der Tauchform im Tauchbad und von der Konzentration der verwendeten Koagulantlösung bestimmt. Bevorzugt ist eine Verweildauer von maximal fünf Minuten. Das Koagulant-Aufbringen 330 kann entweder als erster Herstellungsschritt, vor einem ersten Eintauchen der Tauchform in die Dispersion 311 oder in einer optionalen Verfahrensweise 301, bei Wiederholung 302 der Tauchverfahrensschrittfolge, zwischen zwei Tauchverfahrensschrittfolgen durchgeführt werden.
Der dünnwandige zylindrische Hüllenteil des Urinalkondoms wird nicht unter Verwenden eines Koagulants hergestellt, sondern nur durch - bevorzugt zweimaliges - Betauchen in das Tauchbad aus der Polyurethandispersion und Trocknen der nach dem Herausnehmen der Tauchform aus dem Tauchbad an der Tauchform anhaftenden Dispersion. Die Wanddicke des Hüllenteils beträgt bevorzugt 0,07 bis 0,09 mm, was eine optimale Wasserdampfdurchlässigkeit bei ausreichender mechanischer Stabilität garantiert.

Nach dem Trocknen der Basisschicht und der verstärkten Spitze aus Polyurethan erfolgt das Trennschicht-Aufbringen 321. Dabei wird die Außenseite des penisschaftseitigen zylindrischen Hüllenteils mit Silikon, bevorzugt mittels Sprühnebelauftrag, beschichtet (Coating). Danach erfolgt das Trennschicht-Vernetzen 322, also der chemische Aushärteprozess des Silikons. Nachdem die Vernetzung der Silikonschicht abgeschlossen ist, wird über die Trennschicht eine Klebstoffschicht, bevorzugt Acrylatklebstoff, aufgebracht 323. Dies erfolgt ebenfalls bevorzugt mittels Sprühnebelauftrag.
Abschließend wird das Hüllenteil des Urinalkondoms in Richtung der Spitze aufgerollt 324 und dadurch von der Tauchform abgenommen. Durch das Aufrollen wird die Klebeschicht zwischen der Innen- und der Außenseite des Urinalkondoms positioniert. Beim Abrollen des Urinalkondoms zur Anwendung bleibt die Klebeschicht aufgrund der größeren Haftung des Klebstoffs an Polyurethan als an Silikon an der Innenfläche des Urinalkondoms, also der Basisschicht, haften. Die Klebeschicht steht dann zum Verkleben des Urinalkondoms mit der Haut des Penisschafts zur Verfügung.

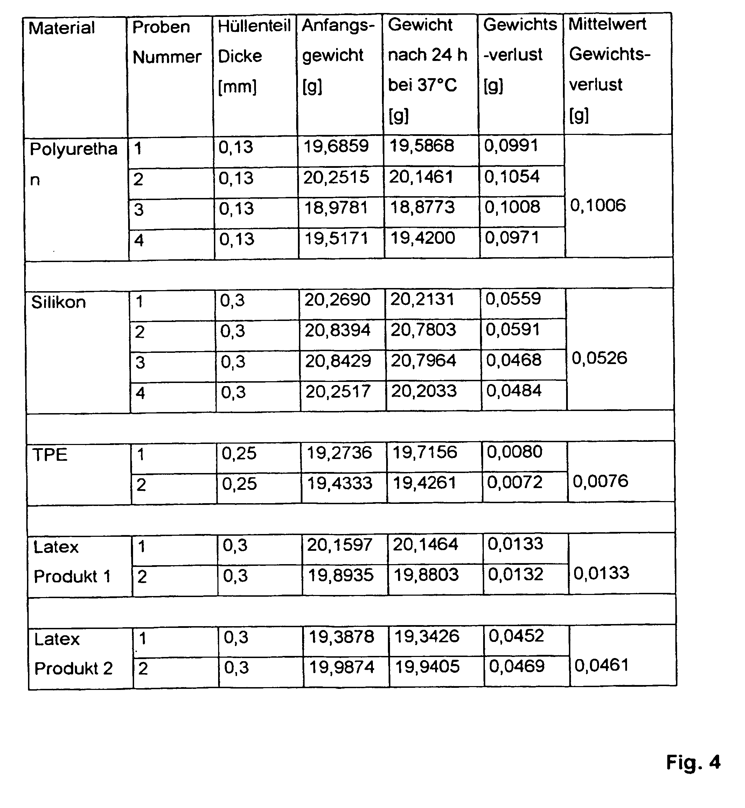

In Fig. 4 ist eine Tabelle bezüglich der Wasserdampfdurchlässigkeit von selbstklebenden Urinalkondomen mit einer Basisschicht aus verschiedenen Materialien dargestellt. Die geprüften Proben bestehen aus drei Schichten: einer Klebstoffschicht, der Basisschicht, deren Material variiert wurde und einer klebstoffabweisenden Trennschicht. Die Probe aus Silikon hat jedoch nur zwei Schichten, da Silikon klebstoffabweisende Eigenschaften hat, ist keine Trennschicht vorhanden. Zum Ermitteln der Messwerte wurden 15 ml Reagenzgläser (Innendurchmesser 15,5 mm) mit 14 ml Wasser gefüllt und mit den aus Urinalkondomen aus unterschiedlichen Materialien ausgeschnittenen Proben abgedeckt, wobei diese seitlich mit einem Spannring verschlossen wurden. Die mit Klebstoff beschichtete Seite der Urinalkondome zeigte dabei zur Wasseroberfläche. Die Proben wurden aus der Mitte des mit Klebstoff beschichteten Hüllenbereichs der Urinalkondome entnommen. Die mit den Proben verschlossenen Reagenzgläser wurden gewogen und anschließend über 24 Stunden in einem Wärmeschrank bei 37 Grad Celsius gelagert, woraufhin die verschlossenen Reagenzgläser erneut gewogen wurden. Der so ermittelte Gewichtsverlust ist ein Maß für die Wasserdurchlässigkeit der Probematerialien. Von den derart überprüften Urinalkondomen weisen die Proben des Urinalkondoms aus Polyurethan die höchste Wasserdampfdurchlässigkeit auf. Die Proben eines Urinalkondoms aus Silikon lassen unter gleichen Bedingungen nur etwa halb so viel Wasserdampf durch. Hinsichtlich des Materials Latex wurden Produkte von zwei verschiedenen Herstellern überprüft. Die Wasserdampfdurchlässigkeiten unterscheiden sich bei den beiden Produkten bei gleicher Wandstärke stark. Sie betragen in einem Fall ca. 45% und im anderen Fall ca. 13% des Wertes der Polyurethanprobe. Die geringste Durchlässigkeit weist ein latexfreies Urinalkondom aus TPE auf, die Durchlässigkeit liegt nur bei ca. 8% des Wertes der Polyurethanprobe. Die Wasserdampfdurchlässigkeit wird neben dem verwendeten Material des Hüllenteils durch dessen Wanddicke bestimmt. Die stark unterschiedlichen Ergebnisse der beiden Latexurinlakondome zeigen, dass neben dem Material der Basisschicht auch die Trennschicht und die Klebstoffschicht die Wasserdampfdurchlässigkeit mitbestimmen.

Vorgeschlagen wird ein Verfahren zum Herstellen eines Urinalkondoms mit einer verstärkten Spitze und einem Hüllenteil, wobei das Urinalkondom mindestens eine Basisschicht aus Polyurethan aufweist und zum Herstellen der Basisschicht und/oder der verstärkten Spitze eine Tauchverfahrensschrittfolge: Bereitstellen eines Tauchbades aus einer wässrigen Dispersion eines aliphatischen Polyurethans, die eine anionische Stabilisierung aufweist, wobei in die Molekülketten des Polyurethans ein anionischer Stabilisator, bevorzugt ein anionisches Tensid, zur anionische Stabilisierung der wässrigen Dispersion, eingebaut ist, Eintauchen einer der gewünschten Urinalform entsprechenden Tauchform in das Tauchbad, Herausziehen der Tauchform aus dem Tauchbad, wobei an der Tauchform eine Schicht der Dispersion anhaften bleibt und Ausbilden einer Polyurethanschicht durch Trocknen der an der Tauchform anhaftenden Dispersion mindestens einmal, für das Herstellen des Hüllenteils bevorzugt zweimal durchgeführt wird.

### Bezugszeichenliste

- 8: Hüllenteil
- 10: Urinalkondom
- 12: Penis
- 14: Basisschichtinnenseite
- 16: konischer Teil
- 18: verstärkte Spitze
- 22: Rohr
- 24: Klebstoffschicht
- 26: Polyurethanbasisschicht
- 28: Trennschicht
- 301: optionale Verfahrensweise
- 302: Tauchverfahrensschrittfolge Wiederholen
- 310: Tauchverfahrensschrittfolge
- 311: Eintauchen der Tauchform in Dispersion
- 312: Herausziehen der Tauchform
- 313: Ausbilden der Polyurethanschicht durch Trocknen
- 320: Endfertigungsschrittfolge
- 321: Trennschicht-Aufbringen
- 322: Trennschicht-Vemetzen
- 323: Klebeschicht-Aufbringen
- 324: Urinalkondom-Aufrollen
- 330: Koagulant-Aufbringen

## Patentansprüche

1. Urinalkondom (10) mit einer verstärkten Spitze (18) und einem Hüllenteil (8), wobei das Urinalkondom (10) mindestens eine Basisschicht aus Polyurethan aufweist und die Polyurethanschicht (26) aus einer eine anionische Stabilisierung aufweisenden wässrigen Dispersion eines Polyurethans hergestellt ist, **dadurch gekennzeichnet, dass**
es sich bei dem Polyurethan um ein aliphatisches Polyurethan handelt, in dessen Molekülketten ein anionischer Stabilisator, bevorzugt ein anionisches Tensid, zur anionischen Stabilisierung der wässrigen Dispersion eingebaut ist.

2. Urinalkondom (10) nach Anspruch 1, **dadurch gekennzeichnet, dass** die wässrige Dispersion weichmacherfrei ist.

3. Urinalkondom (10) nach mindestens einem der Ansprüche 1 bis 2, **dadurch gekennzeichnet, dass** die Spitze (18) eine Verstärkungsschicht aufweist, die durch Verwenden eines Koagulants, bevorzugt einer Calciumnitratlösung aus der wässrigen Dispersion des aliphatischen Polyurethans hergestellt ist.

4. Urinalkondom (10) nach mindestens einem der Ansprüche 1 bis 3, **dadurch gekennzeichnet, dass** das Hüllenteil (8) eine Wandstärke von 0,05 bis 0,1 mm und/oder die Spitze (18) eine Wandstärke von 0,1 bis 0,5 mm aufweist.

5. Urinalkondom (10) nach mindestens einem der Ansprüche 1 bis 4, **dadurch gekennzeichnet, dass** das Hüllenteil (8) mindestens eine klebstoffabweisende Trennschicht und eine Klebstoffschicht (24), bevorzugt aus Acrylatklebstoff, aufweist, wobei die Trennschicht (28) zwischen der Basisschicht (26) und der Klebstoffschicht (24) angeordnet ist und das Hüllenteil (8) in Richtung der Spitze (18) derart aufgerollt ist, dass die Klebstoffschicht (24) zwischen der Trennschicht (28) und der Basisschicht (26) eingerollt ist, wobei die Klebstoffschicht (24) stärker an der Basisschicht (26) als an der Trennschicht (28) anhaftet.

6. Urinalkondom (10) nach Anspruch 5, **dadurch gekennzeichnet, dass** die Trennschicht (28) aus einem Silikonkautschuk, bevorzugt einem Silikonkautschuk mit einer Vernetzungstemperatur unter 100 Grad Celsius besteht.

7. Verfahren zum Herstellen eines Urinalkondoms (10) nach mindestens einem der Ansprüche 1 bis 6 **dadurch gekennzeichnet, dass** zum Herstellen der Basisschicht (26) und/oder der verstärkten Spitze (18) eine Tauchverfahrensschrittfolge, nämlich
(a) Bereitstellen eines Tauchbades aus einer wässrigen Dispersion eines aliphatischen Polyurethans, die eine anionische Stabilisierung aufweist, wobei in die Molekülketten des Polyurethans ein anionischer Stabilisator, bevorzugt ein anionisches Tensid, zur anionische Stabilisierung der wässrigen Dispersion, eingebaut ist,
(b) Eintauchen einer der gewünschten Urinalform entsprechenden Tauchform in das Tauchbad,
(c) Herausziehen der Tauchform aus dem Tauchbad, wobei an der Tauchform eine Schicht der Dispersion anhaften bleibt und
(d) Ausbilden einer Polyurethanschicht durch Trocknen der an der Tauchform anhaftenden Dispersion,
mindestens einmal, für das Herstellen des Hüllenteils (8) bevorzugt zweimal, durchgeführt wird.

8. Verfahren nach Anspruch 7, **dadurch gekennzeichnet, dass** zum Herstellen der verstärkten Spitze (18) der Verfahrensschritt, nämlich
Aufbringen eines Koagulants, bevorzugt einer Calciumnitratlösung im Bereich der Spitze (18) des Urinalkondoms (10) auf die Tauchform vor einer Tauchverfahrensschrittfolge durchgeführt wird, wobei durch das Koagulant eine verstärkte Dispersionsschicht im Bereich der Spitze (18) anhaftet.

9. Verfahren nach Anspruch 8, **dadurch gekennzeichnet, dass** das Koagulant durch Tauchen der Tauchform im Bereich der Spitze (18) des Urinalkondoms (10) in ein Tauchbad aufgebracht wird.

10. Verfahren nach mindestens einem der Ansprüche 7 bis 9, **dadurch gekennzeichnet, dass** nach der Tauchverfahrensschrittfolge eine Trennschicht (28), bevorzugt aus Silikonkautschuk auf die Polyurethanschicht (26) des Hüllenteils (8) des Urinalkondoms (10) und auf die Trennschicht (28) eine Klebstoffschicht (24), bevorzugt durch Sprühnebelauftragen, aufgebracht wird.

11. Verfahren nach Anspruch 10, **dadurch gekennzeichnet, dass** die Trennschicht (28) durch Tauchen des Hüllenteils (8) der Polyurethanschicht (26) auf der Tauchform in ein Silikonkautschukbad oder durch Aufbringen einer Sprühnebelkautschukschicht auf den Hüllenteil (8) der Polyurethanschicht (28) auf der Tauchform aufgebracht wird.

12. Verfahren nach mindestens einem der Ansprüche 10 bis 11, **dadurch gekennzeichnet, dass** für die Trennschicht (28) ein Silikonkautschuk mit einer Vernetzungstemperatur unter 100 Grad Celsius verwendet wird.

13. Verfahren zum Abnehmen des nach mindestens einem der Ansprüche 10 bis 12 hergestellten Urinalkondoms (10) von der Tauchform, **dadurch gekennzeichnet, dass** das Hüllenteil (8) des Urinalkondoms (10) durch Aufrollen des Hüllenteils (8) in Richtung der Spitze (18) von der Tauchform abgenommen wird.

14. Verfahren zum Anwenden des Urinalkondoms (10) nach mindestens einem der Ansprüche 5 bis 6, **dadurch gekennzeichnet, dass** das aufgerollte Hüllenteil (8) des Urinalkondoms (10) über den Penis (12) des Urinalkondombenutzers abgerollt wird, wobei die Klebeschicht (24) an der Basisschicht (26) anhaften bleibt und damit die Basisschicht (26) an der Penishaut angeklebt wird.

## Claims

1. A urinary condom (10) with a reinforced tip (18) and a sheath part (8), with the urinary condom (10) having at least one base layer made of polyurethane and with the polyurethane layer (26) being prepared from an anionically stabilized aqueous dispersion of a polyurethane, **characterized in that** the polyurethane is an aliphatic polyurethane into the molecular chains of which an anionic stabilizer, preferably an anionic surfactant, is incorporated so as to anionically stabilize the aqueous dispersion.

2. The urinary condom (10) according to claim 1, **characterized in that** the aqueous dispersion is free from softeners.

3. The urinary condom (10) according to at least one of the claims 1 or 2, **characterized in that** the tip (18) has a reinforced layer which is prepared from the aqueous dispersion of the aliphatic polyurethane using a coagulant, preferably a calcium nitrate solution.

4. The urinary condom (10) according to at least one of the claims 1 through 3, **characterized in that** the sheath part (8) has a wall thickness of 0.05 to 0.1 mm and/or the tip (18) has a wall thickness of 0.1 to 0.5 mm.

5. The urinary condom (10) according to at least one of the claims 1 through 4, **characterized in that** the sheath part (8) has at least one adhesive-repellent separating layer and an adhesive layer (24), preferably made of acrylate adhesive, with the separating layer (28) being disposed between the base layer (26) and the adhesive layer (24), and with the sheath part (8) being rolled up toward the tip (18) in such a way that the adhesive layer (24) comes to be rolled up between the separating layer (28) and the base layer (26), with the adhesive layer (24) adhering more strongly to the base layer (26) than to the separating layer (28).

6. The urinary condom (10) according to claim 5, **characterized in that** the separating layer (28) consists of a silicone rubber, preferably silicone rubber having a cross-linking temperature of less than 100°C.

7. A method for the production of a urinary condom (10) according to at least one of the claims 1 through 6, **characterized in that** at least one element from the group consisting of the base layer (26) and the reinforced tip (18) is produced by performing a dip molding process at least once, preferably twice for producing the sheath part (8), comprising the following steps:
(a) providing a dipping bath made of an anionically stabilized aqueous dispersion of an aliphatic polyurethane, with an anionic stabilizer, preferably an anionic surfactant, being incorporated into the molecular chains of the polyurethane so as to anionically stabilize the aqueous dispersion,
(b) immersing a dipping form corresponding to the desired urinary shape into the dipping bath,
(c) removing the dipping form from the dipping bath, with a layer of the dispersion adhering to the dipping form, and
(d) creating a polyurethane layer by drying the dispersion adhering to the dipping form.

8. The method according to claim 7, **characterized in that** to produce the reinforced tip (18), the processing step of applying a coagulant, preferably a calcium nitrate solution, to the dipping form in the area of the tip (18) of the urinary condom (10), is carried out prior to the dip molding process, the coagulant causing a reinforced dispersion layer to cling to the area of the tip (18).

9. The method according to claim 8, **characterized in that** the coagulant is applied through immersion of the dipping form in the area of the tip (18) of the urinary condom (10) in a dipping bath.

10. The method according to at least one of the claims 7 through 9, **characterized in that** following the dip molding process, a separating layer (28), preferably made of silicone rubber, is applied to the polyurethane layer (26) of the sheath part (8) of the urinary condom (10), and an adhesive layer (24) is applied to the separating layer (28), preferably by applying an atomizing coating.

11. The method according to claim 10, **characterized in that** the separating layer (28) is applied by dipping the sheath part (8) of the polyurethane layer (26) on the dipping form into a silicone rubber bath, or by coating the sheath part (8) of the polyurethane layer (28) on the dipping form with an atomizing rubber coating.

12. The method according to at least one of the claims 10 to 11, **characterized in that** a silicone rubber with a cross-linking temperature of less than 100°C is used as the separating layer (28).

13. A method for removing the urinary condom (10) produced according to at least one of the claims 10 through 12 from the dipping form, **characterized in that** the sheath part (8) of the urinary condom (10) is removed from the dipping form by rolling the sheath part (8) toward the tip (18).

14. A method for using the urinary condom (10) according to at least one of the claims 5 to 6, **characterized in that** the rolled-up sheath part (8) of the urinary condom (10) is unrolled over the penis (12) of the urinary condom user, with the adhesive layer (24) remaining stuck to the base layer (26) and with the base layer (26) thus being made to adhere to the skin of the penis.

## Revendications

1. Préservatif urinaire (10) avec une pointe renforcée (18) et avec un élément d'enveloppe (8), le préservatif urinaire (10) présentant au moins une couche de base en polyuréthane et la couche en polyuréthane (26) étant fabriquée à partir d'une dispersion aqueuse d'un polyuréthane présentant une stabilisation anionique, **caractérisé en ce que**
le polyuréthane est un polyuréthane aliphatique, dans la chaîne moléculaire duquel est incorporé un stabilisateur anionique, de préférence un agent tensio-actif anionique.

2. Préservatif urinaire (10) selon la revendication 1, **caractérisé en ce que** la dispersion aqueuse est exempte de plastifiant.

3. Préservatif urinaire (10) selon au moins l'une des revendications 1 à 2, **caractérisé en ce que** la pointe (18) présente une couche de renfort, qui est fabriquée par utilisation d'un coagulant, de préférence d'une solution de nitrate de calcium à partir de la dispersion aqueuse du polyuréthane aliphatique.

4. Préservatif urinaire (10) selon au moins l'une des revendications 1 à 3, **caractérisé en ce que** la partie enveloppe (8) présente une épaisseur de paroi de 0,05 à 0,1 mm et/ou **en ce que** la pointe (18) présente une épaisseur de paroi de 0,1 à 0,5 mm.

5. Préservatif urinaire (10) selon au moins l'une des revendications 1 à 4, **caractérisé en ce que** la partie enveloppe (8) présente au moins une couche de séparation anti-adhésive et une couche adhésive (24), de préférence en colle d'acrylate, la couche de séparation (28) étant disposée entre la couche de base (26) et la couche adhésive (24) et la partie enveloppe (8) étant enroulée en direction de la pointe (18), de façon à ce que la couche adhésive (24) soit roulée entre la couche de séparation (28) et la couche de base (26), la couche adhésive (24) adhérant plus fortement sur la couche de base (26) que sur la couche de séparation (28).

6. Préservatif urinaire (10) selon la revendication 5, **caractérisé en ce que** la couche de séparation (28) est en un caoutchouc de silicone, de préférence en un caoutchouc de silicone avec une température de réticulation inférieure à 100 degrés Celsius.

7. Procédé de fabrication d'un préservatif urinaire (10) selon au moins l'une des revendications 1 à 6, **caractérisé en ce que** pour la fabrication de la couche de base (26) et/ou de la pointe renforcée (18), on procède à une séquence de procédés d'immersion, à savoir
a) mise à disposition d'un bain de trempage composé d'une dispersion aqueuse d'un polyuréthane aliphatique, présentant une stabilisation anionique, un stabilisateur anionique, de préférence un agent tensioactif anionique étant incorporé dans la chaîne moléculaire du polyuréthane, pour la stabilisation anionique de la dispersion aqueuse,
b) immersion de l'un des modèles-mères correspondant à la forme souhaitée pour le préservatif urinaire dans le bain de trempage,
c) retrait du modèle-mère du bain de trempage, une couche de la dispersion adhérant sur le modèle-mère et
d) création d'une couche de polyuréthane par séchage de la dispersion adhérant sur le modèle-mère,
au moins une fois pour la fabrication de la partie enveloppe (8), de préférence deux fois.

8. Procédé selon la revendication 7, **caractérisé en ce que** pour la fabrication de la pointe renforcée (17), on réalise l'étape de procédé, à savoir
application d'un coagulant, de préférence d'une solution de nitrate de calcium dans la région de la pointe (18) du préservatif urinaire (10) sur le modèle-mère avant une séquence de procédés d'immersion, une couche de dispersion renforcée adhérant dans la région de la pointe sous l'effet du coagulant (18).

9. Procédé selon la revendication 8, **caractérisé en ce que** le coagulant est appliqué par immersion dans un bain de trempage du modèle-mère, dans la région de la pointe (18) du préservatif urinaire (10).

10. Procédé selon au moins l'une des revendications 7 à 9, **caractérisé en ce qu'**après la séquence de procédés d'immersions, on applique une couche de séparation (28), de préférence en caoutchouc de silicone sur la couche de polyuréthane (26) de la partie enveloppe (8) du préservatif urinaire (10) et une couche adhésive (24) sur la couche de séparation (28), de préférence par application au brouillard de pulvérisation.

11. Procédé selon la revendication 10, **caractérisé en ce qu'**on applique la couche de séparation (28) par immersion de la partie enveloppe (8) de la couche de polyuréthane (26) sur le modèle-mère dans un bain de caoutchouc de silicone ou par application d'une couche de brouillard de caoutchouc sur la partie enveloppe (8) de la couche de polyuréthane (28) sur le modèle-mère.

12. Procédé selon au moins l'une des revendications 10 à 11, **caractérisé en ce que** pour la couche de séparation (28), on utilise un caoutchouc de silicone, avec une température de réticulation inférieure à 100 degrés Celsius.

13. Procédé pour le retrait du préservatif urinaire (10) fabriqué selon au moins l'une des revendications 10 à 12 du modèle-mère, **caractérisé en ce qu'**on retire la partie enveloppe (8) du préservatif urinaire (10) du modèle-mère par enroulage de la partie enveloppe (8) en direction de la pointe (18).

14. Procédé d'utilisation du préservatif urinaire (10) selon au moins l'une des revendications 5 à 6, **caractérisé en ce qu'**on déroule la partie enveloppe enroulée (8) du préservatif urinaire (10) sur le pénis (12) de l'utilisateur du préservatif urinaire, la couche adhésive (24) adhérant sur la couche de base (26) ce qui a pour effet de coller la couche de base (26) sur la peau du pénis.
